# EUROPEAN PATENT SPECIFICATION

(11) **EP 1 979 040 B1**
(45) Date of publication and mention of the grant of the patent: **02.09.2009**
(21) Application number: 07700624.5
(22) Date of filing: 18.01.2007
(51) Int. Cl.: A61N 1/04, A61B 5/04, A61L 24/00, A61B 5/0416, A61N 1/30, A61N 1/18

(54) **IMPROVED BIOMEDICAL ELECTRODE FOR EXTENDED PATIENT WEAR FEATURING A TAP, OR SNAP, WHICH IS ISOLATED FROM THE RETENTION SEAL**
VERBESSERTE BIOMEDIZINISCHE ELEKTRODE ZUR LÄNGEREN ANWENDUNG BEI PATIENTEN MIT EINEM DECKEL ODER SCHNAPPER, DER VON DER HALTEDICHTUNG ISOLIERT IST
ÉLECTRODE BIOMÉDICALE AMÉLIORÉE À PORT PROLONGÉ PAR UN PATIENT COMPORTANT UNE LANGUETTE OU UN FERMOIR, QUI EST ISOLÉE DU JOINT DE RÉTENTION

(30) Priority: 23.01.2006 EP 06100720
(43) Date of publication of application: 15.10.2008
(73) Proprietor: Koninklijke Philips Electronics N.V., 5621 BA Eindhoven (NL)
(72) Inventor: SOLOSKO, Thomas, NL-5656 AA Eindhoven (NL); CROSS, Brett, R., NL-5656 AA Eindhoven (NL); FEUERSANGER, Robert, A., NL-5656 AA Eindhoven (NL); HUGH, Steven, C., NL-5656 AA Eindhoven (NL)
(74) Representative: Schouten, Marcus Maria
(86) International application number: PCT/IB2007/050164
(87) International publication number: WO 2007/083275

(56) References cited:
- WO-A-00/15108
- WO-A-03/002185
- DE-A1- 10 307 446
- DE-U1- 9 316 259
- US-A- 5 938 597

## Description

The present invention relates to a multi-electrode biomedical patch suitable for an extended duration of wear by a person.

In many biomedical applications, electronics and therapy devices need to be attached to the skin in order to observe and monitor patient conditions such as general health, blood flow, heart rhythm, and blood oxygen levels, and they administer therapy as required. Examples of monitoring and therapy devices may include the electrocardiograph (to monitor ECG), external defibrillators or pacing devices, nerve stimulation devices, and transdermal drug delivery systems. In some applications, these electronics and therapy devices need to be attached for extended periods of time.

Bio-medical monitoring patches may be designed to contain a single electrode, such as a snap-style ECG electrode, or multiple electrodes, like the Philips Medical Systems' Cardiac Monitoring Patch. In these kinds of a bio-medical patch, a hydrogel makes direct contact with the skin in two or more locations. This hydrogel improves the electrical conductivity between the silver/silver chloride layer making up the actual electrode and the skin. Typical components of a conductive hydrogel include water (which acts as the solvent), water-soluble monomers, which crosslink to give structure to the gel and which may also provide skin adhesion, humectant materials which reduce the dryout characteristics of the hydrogel, and electrolytes or salts such as sodium chloride or potassium chloride dissolved in water, which provide the ionic conductivity. One advantage of hydrogels over other conductive electrolytes is that they can be removed cleanly from the skin without leaving a residue.

The silver/silver chloride (Ag/AgCl) electrode layer contacts the hydrogel on one side, and the silver or copper traces on the other side. The electrode layer is the interface at which ionic conduction through the hydrogel changes to electronic conduction to the monitoring or therapy device. The traces, which may be printed or etched, provide electrical connection between the electrode and the monitoring device via the electro-mechanical connector. This device is typically a high impedance device, which provides an effectively open circuit between electrodes and prevents current from flowing between them.

More in detail, each electrode is composed of the Ag/AgCl layer and the hydrogel layer, which contacts the skin. To separate the electrodes and ensure they don't touch each other, a dielectric or barrier material is needed to fill in the gaps between each electrode. In addition, a thin, flexible, breathable retention material is required to hold the patch to the patient's skin, and to protect the electrodes from external liquid entry, such as shower water. All these materials may touch the skin. Depending upon where they are located in the patch laminate, they will most likely create an uneven skin-contacting surface with dints, voids and protrusions.

Many current biomedical electrodes are made to stick well to the skin for several hours, but are not optimized for multi-day patient comfort. The materials that are used, such as polyethylene foam coated with medical grade adhesive, and vinyl film, are occlusive to moisture vapor and do not allow body moisture to escape. Trapped moisture quickly irritates and damages the skin. In addition, these materials are not very flexible and restrain the skin, causing additional discomfort. Also, the surface of many electrodes is not flat. Often, a silver/silver chloride snap is attached to the center of the electrode, covered with hydrogel and surrounded with adhesive-coated foam. Since the skin stretches under a constant load, the uneven design of these electrodes causes the skin to stretch under the raised snap area. Stretched skin also becomes irritating after a few day.

Patient comfort is paramount in a multi-day biomedical electrode patch design. Not only must it be breathable and allow body moisture to escape, but all skin contacting materials must be flexible and non-irritating. In addition, the electrode or electrode patch should be designed so that the skin interface is smooth, flat and even.

Also, some patients are sensitive or may become sensitive to the adhesive materials applied to the skin. In order to minimize the chance of irritation or sensitization, biomedical electrodes should be designed to minimize the number of different materials in contact with the skin.

Another challenge experienced by many snap-style ECG electrode designs is their tendency to buckle on the skin under the sheer load caused by the weight of the lead wires pulling the snap downwards. In these electrode designs, the snap is attached directly to the skin-contacting layer. Therefore, sheer forces may cause voids between the electrode and the skin as the electrode buckles. The creation of voids between the electrode and the skin causes an increase in skin impedance (due to smaller skin contact area), additional noise in the ECG, and the potential for the skin to stretch and creep uncomfortably into the voided spaces. Partially contacting electrodes may also pull uncomfortably at the skin. In some cases, these sheer forces are great enough to peel the electrode partially or completely off the skin. This can happen with tab-style electrodes.

A biomedical patch or electrode assembly is needed that is designed to optimize and enhance patient comfort for multi-day application by creating a stable, smooth, and even skin interface while minimizing the number of different materials in contact with the skin. Furthermore, improvements in comfort, reliability, and stability of snap and tab-style ECG electrodes are needed.

It is an object of the invention to provide an extended wear biomedical electrode assembly, comprising:
at least two conductive gel pads spaced apart from each other,
electrodes in contact with the gel pads,
a support construction disposed substantially laterally with respect to the gel pads, surrounding the gel pads, filling space between neighboring gel pads, and overlapping an lower, outer perimeter of each of the gel pads,
wherein said support construction is thinner than the gel so that the gel is gently pushed through the support construction onto the skin ensuring good hydrogel to skin contact.

An advantage of the proposed assembly is that the relative position of the two or more electrodes is well defined. The gel pads may have a substantially flat configuration, for example disk-shaped. In this case and similar cases, the gel pads may be aligned by their skin contacting faces to the skin surface. Especially for the lower face, which eventually makes contact with the skin of a user, this ensures that all pads are evenly applied to the skin. For ease of explanation, the direction from electrodes to skin is assumed to be the up-down direction. This is not to be construed to any particular orientation of the electrode assembly. In fact, the electrode assembly may be applied in any orientation. A spacing between two electrodes of any pair of electrodes is needed for electrical insulation of one electrode to the other. Another reason is that different electrical signals from different regions beneath the electrode assembly can be collected.

The support configuration, or frame, is advantageous in that it holds the gel pads in place. In the case of the gel pads having a flat or disk-like configuration, the support configuration extends substantially in the space that is defined by expanding the gel pads in radial directions. The skin contacting surface of the support configuration substantially reproduces the shape of the underlying skin.

Furthermore, the support configuration is advantageous since it ensures a reliable fixation of the electrode assembly to the skin. Especially for multi electrode assemblies having a relatively large area, sheer forces and torques may act on the electrode assembly. In particular, long lever arms presented by the electrode assembly amplify forces that are applied to the electrode assembly for example by the electro-mechanical connector. A more even distribution of the occurring forces across the contact surface prevents localized force peaks to appear.

Another advantage of the proposed support configuration is that it supports the gel pads in the up-down direction (perpendicular to the surface of the skin) since it overlaps an outer perimeter of each of the gel pads. If a thin support configuration is provided, the perimeter of a gel pad overlapped by the support configuration may be substantially in the same plane as the major portion of the lower surface of the corresponding gel pad. If the support configuration has a considerable thickness, a beveled or cascaded configuration may be provided at the transition between support configuration and gel pad. This combines a flat, smooth transition with supporting the gel pad in the up-down direction.

The proposed electrode assembly mechanically isolates the snap or tab from the skin-contacting support configuration. The snap or tab connections in this invention are linked to the top of the support configuration which helps to isolate snap or tab movement from its lower side, which adheres to the skin. This isolation helps to maintain full electrode-to-skin contact, thus improving electrode performance and patient comfort. This isolation also helps prevent the support configuration (and therefore the electrodes) from peeling prematurely away from the skin.

In one embodiment, the connection with a monitoring device comprises an electro-mechanical connector electrically connected to said electrodes.

An electro-mechanical connector is advantageous in that it allows connection directly to a monitoring device. The electro-mechanical connector is configured to allow temporary disconnection of the electrode assembly from the lead connecting it to a monitor or therapy device, for example, when the user wants to recharge his monitoring/therapy device, or replace a battery in the current device. Biomedical monitoring and/or therapy delivery is interrupted during these periods, but the increased comfort of use for the user makes up for it in most applications. Upon reconnecting the monitoring device to the electrode assembly, biomedical monitoring and/or therapy delivery may continue. Since the electrode assembly remains on the body, the electrode assembly is still in the same position so that long term monitoring is performed under unchanged conditions. In a therapy delivery scenario, an advantage is that a physician may place the electrode assembly using his expert knowledge so that it remains at this well defined position throughout the period of use, which may be several days or a week.

In the area where the electro-mechanical connector is attached, the support configuration usually has a planar configuration due to the interaction with the electro-mechanical connector. However, if the intended use of the electrode assembly is known in advance, the electromechanical connector may be provided with an appropriate shape, as well. In particular, the electro-mechanical connector may present a convex shape in one or two directions.

The electro-mechanical connector may be electrically connected to the electrodes via at least one conducting path per electrode, each conducting path extending from a corresponding electrode to the electro-mechanical connector.

An advantage of a conducting path per gel pad to the electro-mechanical connector is that a signal may be collected from or applied to each individual gel pad. A conducting path may comprise for example an Ag/AgCl electrode and a strip conductor disposed in a printed or etched circuit layer of the electrode assembly.

In a further embodiment, the support configuration of an extended wear biomedical electrode assembly comprises:
a support substrate disposed substantially laterally with respect to the gel pads, surrounding the gel pads and filling gaps between neighboring gel pads,
at least one retention seal attached to a lower face of the support substrate opposite to a connector facing side thereof, the retention seal overlapping an outer perimeter of each of the gel pads.

By separating the support configuration in (at least) two distinct elements, the different functionality of the support configuration can be distributed to the elements. This is advantageous in that each element may be of an appropriate material. For example, the retention seal may be made of a breathable material. The same holds for the desired size of each of the elements.

Good hydrogel to skin contact is advantageous for improved electrical conductivity between their appropriate surfaces. It is also advantageous in that no recess is created at the site of the hydrogel. A recess would cause the skin to slightly protrude within the electrode assembly. Over the utilization period of the electrode assembly, this may lead to the skin also protruding into small gaps between the hydrogel and the retention seal and/or the support substrate.

In an alternative embodiment of the present invention, the support configuration comprises a retention seal disposed substantially laterally with respect to the gel pads, surrounding the gel pads, filling space between neighboring gel pads, and overlapping a lower, outer perimeter of each of the gel pads.

The advantages recited for the preferred embodiment are also valid for this embodiment. The difference is that the retention seal also assumes the functionality of the support substrate. In other words, the retention seal assumes all the functionality associated with the support configuration. To this end, the retention seal consists of an appropriate material providing conformability, breathability in the up-down direction, and sealing properties in a radial direction. Again, if the retention seal presents a considerable thickness, then it may be advantageous to bevel or cascade the transition from the retention seal to the gel pad. This assures a reliable support of the gel pad in the up-down connection.

In a related embodiment, the retention seal comprises a substrate at the most as thick as the hydrogel pads disposed substantially laterally with respect to the gel pads, surrounding the gel pads and filling space between neighboring gel pads, and at least one additional thin layer attached to a lower face of said retention seal opposite to a connector facing side thereof, this additional thin layer overlapping an outer perimeter of each of the gel pads.

In accordance with an embodiment of the present invention, the gel pads comprise a cured hydrogel. A cured hydrogel has the advantage of a longer shelf life compared to liquid hydrogels dispensed in a foam matrix. Another advantage is that it may be formed in a desired manner.

The cured hydrogel is a piece cut from a pre-cured sheet, or is a part that is cast and cured in place. The advantage of using a piece cut from a pre-cured sheet is a facilitated manufacturing. If the supplier of the pre-cured sheets guarantees constant properties of the pre-cured sheets, this also holds for a plurality of gel pads fabricated from these. The advantage to dispensing and curing the gel directly into the wells provided by either the support substrate or the retention seal is that it can be cured flush with the top surface of the retention seal. This forms a perfectly smooth and even skin-contacting surface. Curing may be performed by e.g. UV curing. Under certain conditions it may be contemplated to use a piece from a pre-cured sheet as a core and to cast additional gel around it.

The support substrate may consist of foam having dielectric properties. This provides the required dielectric barrier between the electrodes. As such, the dielectric support substrate also acts as an insulator.

The conducting paths may be passed through a circuit layer bonded to the electro-mechanical connector on one side and to the support substrate on said other side. This is advantageous in that the conducting paths are pre-defined. In the case of a multi electrode assembly, the conducting paths run orthogonal to the up-down direction, at least for a portion of their length, in order to be gathered at the electro-mechanical connector.

The retention seal may extend around a lateral face of said support substrate, a layer comprising the conducting paths, and a perimeter of said electro-mechanical connector. In this manner, the retention seal, the lower surface of the gel pads, and the electrical connector form a housing for the electrode assembly. An advantage is the improved sealing property of the entire assembly. By.using an appropriate material for the retention seal, the retention seal may be water-proof, but permeable for vapor and air.

In a further preferred embodiment according to the present invention, a medical monitoring apparatus or a medical therapy delivery apparatus is provided. This apparatus comprises an extended wear biomedical electrode assembly as described above. An advantage is that the electrode assembly and the apparatus may be matched one to each other. This matching may include mechanical and electrical matching, and also an appropriate calibration. The electrode assembly may be detachable from the apparatus.

In another preferred embodiment of the present invention, a method for applying a biomedical electrode assembly as described above comprises the steps of providing the biomedical electrode assembly, and affixing the biomedical electrode assembly to a patient.

If the biomedical electrode assembly comprises a peelable protective sheet, this protective sheet may be removed from the lower face of the support construction prior to affixing the biomedical electrode.

An advantage of providing a protective sheet that is removed prior to affixing the biomedical electrode assembly is a longer shelf time of the biomedical electrode assembly. Another advantage is the ease of use of the biomedical electrode assembly. Alternatively, the adhesive may also be applied to the electrode assembly immediately before affixing the electrode assembly.

Features and advantages of the invention will become apparent from the following description of preferred embodiments of the invention, given by way of example only and made with reference to the accompanying drawings. The accompanying drawings are not necessarily to scale.
Fig. 1 is an exploded perspective view of a biomedical multi-electrode assembly according to a first embodiment of the present invention.
Fig. 2 is a perspective view of the electrode assembly of Fig. 1 attached to the skin of a user.
Fig. 3 is a sectional view of the electrode assembly according to a first embodiment of the invention along the line III-III of Fig. 4.
Fig. 4 is a sectional view of the electrode assembly according to a first embodiment of the invention along the line IV-IV in Fig. 2.
Fig. 5 is an exploded perspective view of an electrode assembly according to a second embodiment of the present invention.
Fig. 6 is a perspective view of the electrode assembly of Fig. 5 attached to the skin of a user.
Fig. 7 is a sectional view of the electrode assembly according to a second embodiment of the invention along the line VII-VII of Fig. 6.
Fig. 8 is an exploded perspective view of an electrode design according to a third embodiment of the present invention.
Fig. 9 is an exploded perspective view of an electrode design according to a fourth embodiment of the present invention.

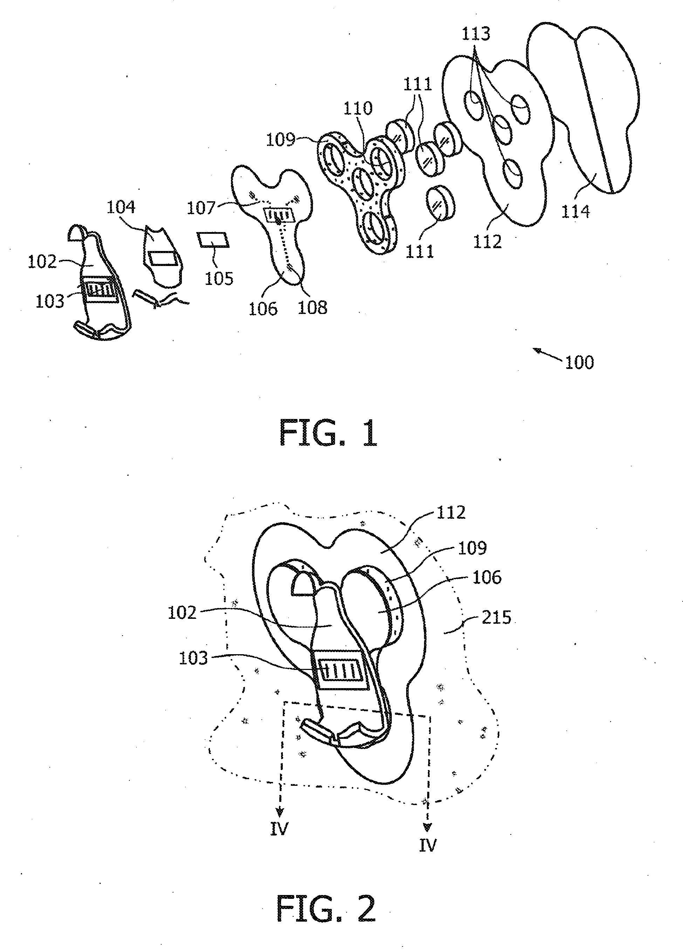

Fig. 1 shows a biomedical electrode assembly 100, also called biomedical electrode patch.

An electro-mechanical connector 102 provides the mechanical and electrical connection between the patch and a monitoring or therapy delivery device. Conductive contacts 103 (such as conductive silicone contacts) on the clip make electrical connection with conductive contacts on the patch. The patch contacts can be printed silver or carbon. The electro-mechanical connector 102 is bonded to the remaining patch by an adhesive 104 such as a pressure sensitive adhesive (PSA). Adhesive 104, which should be thin, provides a water-tight seal around each electrical contact of the patch. It may be for example 3M's 1524 medical grade PSA.

A piece of z-axis electrically-conductive PSA 105 ensures a connection between the clip contacts and the printed contact areas of the patch. It conducts only through its thickness. If it is a structural adhesive as well, it may be enlarged and used in place of adhesive 104. Alternatively, if the connection between electro-mechanical connector and patch is acceptable without the aid of this layer of PSA 105, it may be eliminated.

A printed polyester circuit layer 106 conducts signals from the electrodes to the contacts 103 of electro-mechanical connector 102. In this embodiment, the circuit 107 is printed on both sides of the polyester film. The side facing electro-mechanical connector 102 is printed with conductive silver ink that is connected via printed thru-holes to the silver/silver chloride biomedical electrodes 108 on the patient side.

The electrode assembly also comprises a support substrate 109. This 17 - 50 mil (0.4 - 1.3 mm) foam layer holds individual hydrogel pads 111 in wells 110. The support substrate 109 provides the required dielectric barrier between electrodes. It may be the same nominal thickness as the hydrogel pads 111. Alternatively, it may also be a little thinner to push the hydrogel pads 111 gently through openings 113 of a retention seal 1 l 2..

The conductive hydrogel pads, one for each electrode, provide the ionic conduction between the electrode and the skin. A hydrogel pad 111 captures the body voltages and conducts them to the corresponding electrode 108. Because they are electrically isolated from each other, each piece creates an independent electrode when assembled to the monitoring device.

The conductive hydrogel, such as Axelgaard's Ag602, may be supplied in pre-cured sheets, or dispensed or cast and cured in place with processes such as UV curing. The advantage to dispensing and curing the gel directly into the wells 110 provided by the support substrate 109 is that it can be cured flush with the top surface of the retention seal. This forms a perfectly smooth and even skin-contacting surface.

The retention seal 112 is a thin, flexible, breathable layer such as 1-mil (0.025 mm) Polyurethane (PU) film or 5 - 10 mil (0.125 - 0.25 mm) PU foam, coated on one side with medical grade pressure sensitive adhesive. The pressure sensitive adhesive holds the patch to the skin, while the thin substrate allows skin moisture to evaporate and seals against outside moisture from entering and causing electrical shorting between electrodes.

The four large holes 113 through this layer are the windows through which the hydrogel makes contact with the skin. These holes also define the center-to-center spacing of the four electrodes. Instead of providing four electrodes, the invention may also be carried out with two or more electrodes.

During the storage period prior to the actual use of the biomedical patch, a peelable protective sheet 114 is provided that covers the pressure sensitive adhesive of the retention seal 112 and keeps the hydrogel pads from losing moisture and drying out.

Fig. 2 shows the electrode assembly or biomedical patch when applied to an area of the skin 215 of a user or patient. The large, breathable retention seal 112 provides a secure, yet comfortable fixation of the electrode assembly to the skin. The support substrate 109 decouples the electro-mechanical connector 102 from the retention seal 112 to some extent. In particular, when the electro-mechanical connector is tilted or skewed in response to a traction force caused by a monitoring device connected to the electro-mechanical connector 102, the support substrate 109 is capable of moderating a corresponding effect for the retention seal 112.

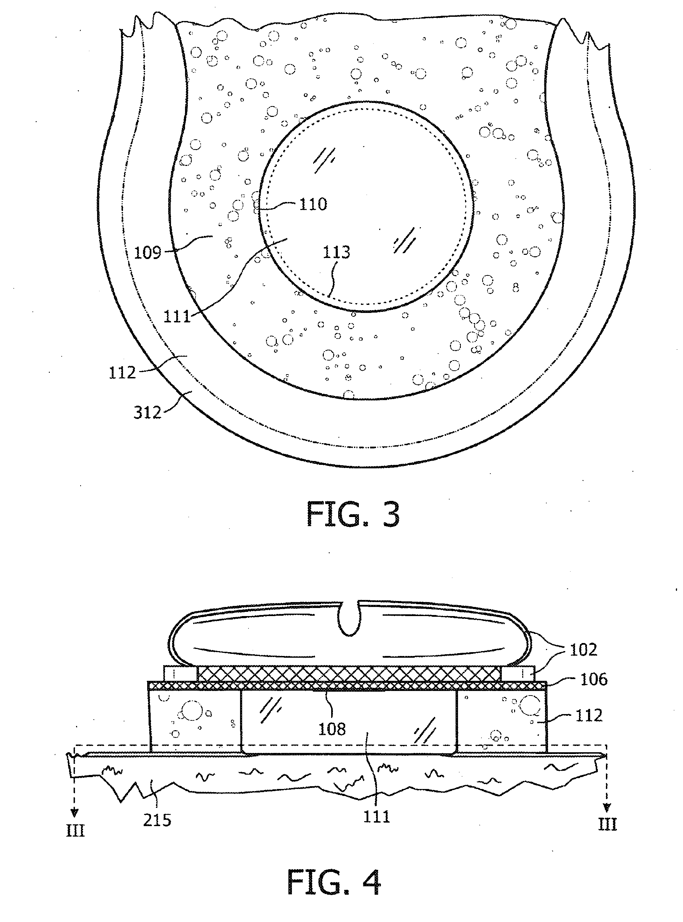

Fig. 3 shows a section of the electrode assembly of Figs. 1 and 2 in a plane substantially parallel to the plane of the skin. The view is directed downwards, that is looking in the direction from the electro-mechanical connector (not shown) to the skin. The location and orientation of the skin can be seen in Fig. 4.

In Fig. 3, it can be seen that the hydrogel pad 111 is disposed in a well or through-hole 110 of the support substrate 109. Preferably, the support substrate firmly surrounds the hydrogel pad. The retention seal 112 is situated beneath the support substrate 109 and the hydrogel pad 111. Retention seal 112 also presents an outer area 312. As explained above, such an outer area is important for the fixation of the patch to the skin.

In Fig. 4 it can be observed that the retention seal performs several functions in addition to fixing the patch firmly to the skin 215. First, the diameter of the holes 113 in the retention seal may be smaller than the diameter of the gel pieces 111. Having smaller holes, the retention seal overlaps the edges of all the gel pieces 111, thus holding them in place in the support substrate 109 against the respective electrodes 108. This overlap performs one additional function - it eliminates the spaces that would otherwise exist around each gel pad 111 between the outside gel and the inside of the support substrate. Without this overlapping retention seal, the skin would tend to flow into and fill this gap. This would create a raised edge of skin around each gel piece and potentially become irritable and uncomfortable. The retention seal covers these gaps and creates a smooth skin-contacting surface.

Since the retention seal does add thickness to the skin-contacting surface of the patch, it is important that the hydrogel be pushed gently through the thickness of the retention seal to maintain a smooth, flat skin-contacting surface. This pushing can be achieved by using a support substrate that is thinner (0.010 - 0.015 inch thinner) than the hydrogel. Since the side of the support substrate 109 which faces the electro-mechanical connector 112 is bonded to the circuit layer 106, the hydrogel 111 has no place to move but through the holes 113 in the retention seal 112.

The overlapping retention seal 112 also prevents the support substrate from touching the skin. This decreases the number of skin-contacting patch materials from three to two, which decreases the risk that a patient will react to one or more of the patch materials.

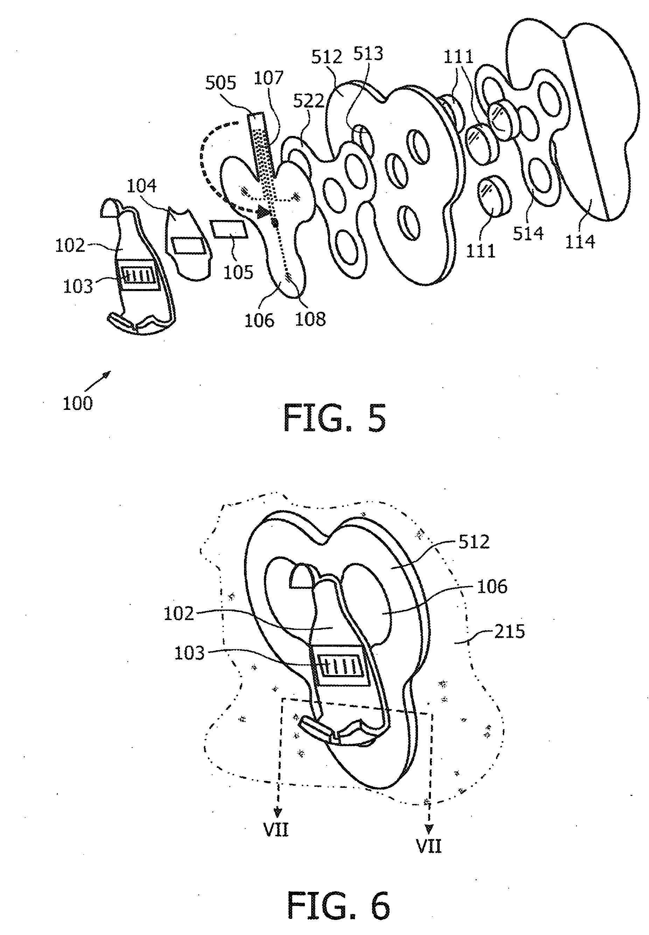

Fig. 5 shows an electrode assembly or biomedical patch according to another embodiment of the present invention in an exploded perspective view.

The biomedical patch shown in Fig. 5 comprises six layers.

The electro-mechanical connector 102 provides the mechanical and electrical connection between the patch and the monitoring device (monitoring device not shown). The electrical connection is provided by conductive contacts 103.

The adhesive 104 for the electro-mechanical connector 102 bonds the electro-mechanical connector to the top of the remaining stack of the biomedical patch. This adhesive should be thin, like 3M's 1524, 2.5-mil medical grade PSA.

A piece of z-axis electrically-conductive PSA 105 ensures a connection between the clip contacts and the printed contact areas of the patch. It conducts only through its thickness. If it is a structural adhesive as well, it may be enlarged and used in place of adhesive 104. Alternatively, if the connection between electro-mechanical connector and patch is acceptable without the aid of this layer of PSA 105, it may be eliminated.

A printed polyester circuit layer 106 conducts signals from the electrodes 108 to the contacts 103 of electro-mechanical connector 102. In this embodiment, the circuit 107 is printed on one side of the polyester film. The traces are brought up the length of a tab 505 which is creased and folded so that it can be bonded to the underside of a tongue (not shown) of the electro-mechanical connector 102.

A pressure sensitive adhesive (PSA) 522 for the retention seal bonds the circuit layer 106 to the top of the retention seal 512 and also provides the moisture seal , around each printed electrode. A 2.5-mil adhesive such as the 3M 1524 transfer adhesive is proposed as one among several possibilities.

There is also an optional gel retainer layer 514 on the skin-contacting lower face of the retention seal 512. This is a thin film (such as 1-mil Polyurethane) coated both sides with medical-grade PSA. The gel retainer layer has holes axially aligned with the hydrogel pads 111. The diameters of the holes in the gel retainer layer are smaller than the diameter are smaller than the diameter of the gel pieces. This holds the gel pieces in place in the retention seal during wear and upon removal.

The conductive hydrogel pads, one for each electrode, provide the ionic conduction between the electrode 108 and the skin 215. A hydrogel pad 111 captures the body voltages and conducts them to the corresponding electrode 108. Because they are electrically isolated from each other, each piece creates an independent electrode when assembled to the monitoring device.

The conductive hydrogel, such as Axelgaard's Ag602, may be supplied in pre- - cured sheets, or dispensed or cast and cured in place with processes such as UV curing. Dispensing and curing the gel directly into the wells 513 provided by the retention seal 512 is advantageous in some aspects. It can be cured right to the top surface of the retention seal forming a perfectly smooth and even skin-contacting surface. Dispensed gel also allows the retention seal to become as thin and flexible as practical and desired. There may be a minimum hydrogel thickness required to ensure a complete fill. If that thickness is 5 - 10 mil (0.125 - 0.25 mm), the retention seal can become very thin. If more gel is desired, i.e. to increase shelf life, the retention seal thickness can be chosen to be as thick as the hydrogel can be reliably cured (potentially 50 mil and greater).

In the described embodiment, the retention seal 512 may be fairly thick (10 - 30 mil, corresponding to 0.25 - 0.75 mm) such as Smith and Nephew's Allevyn material. The retention seal 512 can be the same thickness as the sheet or dispensed hydrogel thus presenting a smooth, even surface to the skin. It cannot be thicker than the hydrogel pads 111, since this would prevent the hydrogel from touching the skin.

The four large holes 513 through this layer are the gel wells which hold the hydrogel and enable them to make contact with the skin. These holes also define the center-to-center spacing of the four electrodes.

Because the gel wells 513 are located in the retention seal 512 in this embodiment, the need for a separate support substrate is eliminated. This simplifies the patch design. It also creates a patch with a more homogeneous flexibility. The support substrate, being more rigid than the flexible retention seal in the previous embodiment, creates a region where the skin is not allowed to stretch as freely as the rest of the patch. This more rigid area may create some skin irritation during wear or upon removal as the skin flexes differently under different areas of the patch. The thicker retention seal cushions the skin from the more rigid upper layers (circuit 107 and connector 102) and provides a uniform skin-flexing surface.

One side of this material is or may be coated with an adhesive such as a medical grade pressure sensitive adhesive PSA. The PSA holds the patch to the skin, and prevents outside moisture from entering and causing electrical shorting between electrodes. If a self-adhesive material, such as Smith & Nephew Allevyn foamed polyurethane, is used, no additional PSA is required.

This invention may also be made with two or more large holes 513 in the retention seal 512.

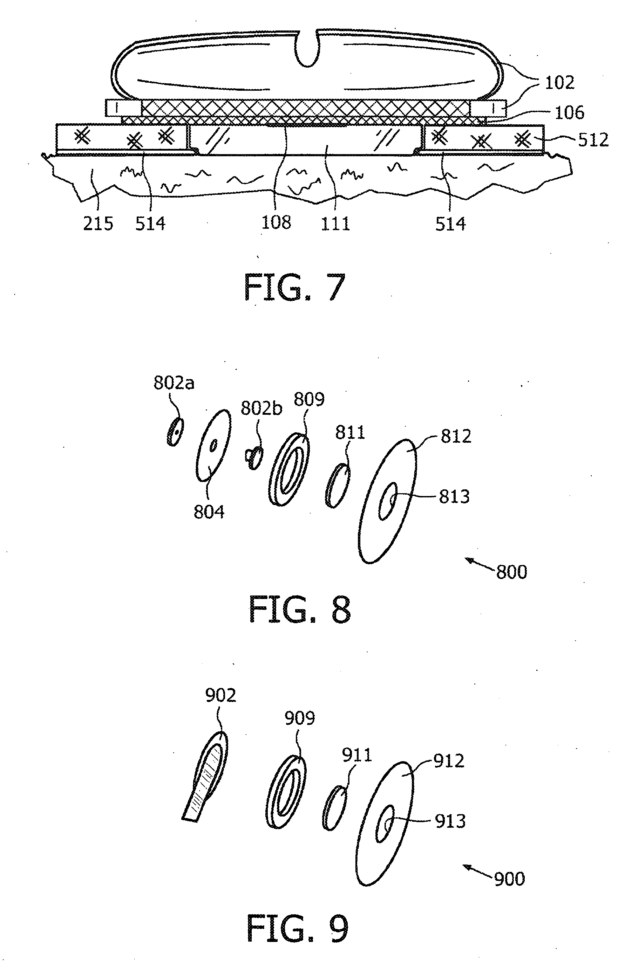

It may further be contemplated to adopt the present invention to single snap-stype biomedical electrodes with a smooth, even skin-contacting surface. Such an electrode 800 represented in Fig. 8 would also be suited for long-term wear.

The snap is composed of an Ag/AgCl eyelet 802b inserted through a stable support material 804, such as 1-mil polyester film. The center of stable support material 804 shows a hole where the eyelet post is inserted. The eyelet's post is pressed into the metal end of the snap cap 802a which secures it to the substrate.

The support 804 should be strong enough to hold the snap and prevent it from tearing out, but thin enough to maintain electrode flexibility.

The flexible support substrate 809, made from a material such as polyurethane foam coated on both sides with PSA, may be similar in thickness to the eyelet + hydrogel thicknesses. It is bonded to the thin support film 804 on one side and to the retention seal 812 on the other. The hydrogel 811 is placed inside the support substrate in contact with the Ag/AgCl snap. It may be sheet gel, such as Axelgaard's Ag 602, or dispensed gel that is cured in place. Again, there is an advantage to dispensing and curing the gel directly into the wells provided by the support substrate. Dispensed gel can be cured right to the top of the retention seal forming a perfectly smooth and even skin-contacting surface.

As before, the retention seal 812 is a breathable material (such as a 1-mil PU film or 5-10 mil PU foam) which holds the electrode firmly against the skin. The diameter of the single hole 813 in this layer is smaller than the diameter of the hydrogel 811, thus trapping the hydrogel in place inside the foam ring and creating a smooth skin-contacting surface. This design also eliminates contact between the foam ring 809 and the skin, thus minimizing the number of different skin contacting materials.

Many current electrodes rely upon the foam ring to provide the skin adhesion. Consequently, it is relatively large compared to the diameter of the hydrogel it surrounds. This ring is often thick and occlusive, and does not allow the skin to breath. Consequently, for long-term wear, body moisture is trapped under this ring and becomes irritating and uncomfortable. Also, thicker materials may catch clothing and peel away from the skin more easily than thin materials.

Using a breathable retention seal to hold the electrode to the skin in place of the support substrate allows the support substrate to become smaller. This decreases the moisture-occlusive area of the electrode, increases electrode breathability, and reduces the chance of catching an edge and inadvertently peeling the electrode away from the skin. It also increases the flexibility of the electrode in that the dimensions of the stiffer support substrate layer are minimized.

Another single bio-medical electrode design 900 shown in Fig. 9 incorporating the same design features to improve comfort for long-term wear differs from the single electrode design described with reference to Fig. 8 in the following features. In place of the snap, the stable supporting substrate 902, such as 1-mil polyester, is printed with an electrode reagent, such as Ag/AgCl ink, on the patient side. This layer should again be as thin and flexible as possible.

The flexible foam ring 909, made from a material such as polyurethane foam coated on both sides with a PSA, may be similar in thickness to the hydrogel 911. It is bonded to the thin printed substrate layer 902 on one side and to the retention seal 9112 on the other. The hydrogel is placed inside the foam ring in contact with the printed Ag/AgCl electrode. It may be sheet gel, such as Axelgaard's Ag602, or dispensed and cured in place.

The retention seal is again a breathable material (such as 1-mil PU film or 8-mil PU foam) which holds the electrode firmly against the skin. The diameter of the single hole 913 in this layer is smaller than the diameter of the hydrogel 911, thus trapping the hydrogel in place inside the foam ring 912, and creating a smooth skin-contacting surface. This design also eliminates contact between the foam ring 912 and the skin, thus minimizing the number of different skin contacting materials.

The embodiments depicted and described herein are meant to be illustrative in nature, and it will be understood that various biomedical electrode assemblies or patches may be designed using the principles set forth herein, and used for various commercial and consumer applications.

## Claims

1. Extended wear biomedical electrode assembly (100), comprising:
at least two conductive gel pads (111) spaced apart from each other,
electrodes (108) in contact with the gel pads (111),
a support construction (109, 102) disposed substantially laterally with respect to the gel pads (111), surrounding the gel pads, filling space between neighboring gel pads, and overlapping an outer perimeter of each of the gel pads on the lower side of the biomedical electrode assembly which is intended to contact skin,
wherein said support construction (109, 102) is thinner than the gel so that the gel is gently pushed through the support construction onto the skin ensuring good hydrogel to skin contact.

2. Extended wear biomedical electrode assembly according to claim 1, comprising an electro-mechanical connector (102) electrically connected to said electrodes (108) for connection with a monitoring device.

3. Extended wear biomedical electrode assembly according to claim 2, wherein the electro-mechanical connector (102) is electrically connected to said electrodes (108) via at least one conducting path per electrode, each conducting path extending from a corresponding electrode to said electro-mechanical connector.

4. Extended wear biomedical electrode assembly according to any one of claims 1 to 3, wherein said support construction (109, 112) comprises:
a support substrate (109) disposed substantially laterally with respect to the gel pads, surrounding the gel pads and filling space between neighboring gel pads,
at least one retention seal (112) attached to a lower face of said support substrate opposite to a connector facing side thereof, the retention seal overlapping an outer perimeter of each of the gel pads.

5. Extended wear biomedical electrode assembly according to any one of claims 1 to 3, wherein said support construction (109, 112) comprises a retention seal (112) disposed substantially laterally with respect to the gel pads (111), surrounding the gel pads, filling space between neighboring gel pads, and overlapping an lower, outer perimeter of each of the gel pads.

6. Extended wear biomedical electrode assembly according to claim 5, wherein said retention seal (112) comprises:
a substrate at the most as thick as the hydrogel pads disposed substantially laterally with respect to the gel pads, surrounding the gel pads and filling space between neighboring gel pads,
at least one additional thin layer attached to a lower face of said retention seal opposite to a connector facing side thereof, this additional thin layer overlapping an outer perimeter of each of the gel pads.

7. Extended wear biomedical electrode assembly according to any one of claims 1 to 6, wherein said gel pads (111) comprise a cured hydrogel.

8. Extended wear biomedical electrode assembly according to claim 7, wherein the cured hydrogel is a piece cut from a pre-cured sheet, or is a part that is cast and cured in place.

9. Extended wear biomedical electrode assembly according to any one of claim 1 to 8, wherein said support construction (109, 112) comprises foam having dielectric properties.

10. Extended wear biomedical electrode assembly according to any one of claims 1 to 9, wherein said conducting paths are passed through a circuit layer (106) bonded to the electro-mechanical connector (102) on one side and to said support construction (109, 112) on said other side.

11. Medical monitoring and/or therapy delivery apparatus comprising an extended wear biomedical electrode assembly (100) according to any one of claims 1 to 10.

12. Method for applying the biomedical electrode assembly (100) according to any one of claims 1 to 11, the method comprising the steps of
providing said biomedical electrode assembly (100), and
affixing said biomedical electrode assembly (100) to a patient.

13. Method according to claim 12, further comprising the step of removing a protective sheet from the lower face of said support construction (109, 112) prior to affixing said biomedical electrode assembly (100).

## Patentansprüche

1. Biomedizinische Elektrodenbaugruppe (100) zur längeren Anwendung, die Folgendes umfasst:
mindestens zwei leitende Gelpads (111) in einem Abstand voneinander,
mit den Gelpads (111) in Kontakt befindliche Elektroden (108),
eine Trägerkonstruktion (109, 112), die im Wesentlichen seitlich in Bezug auf die Gelpads (111) angeordnet ist, die Gelpads umgibt, den Raum zwischen benachbarten Gelpads auffüllt und einen Außenumfang von jedem der Gelpads auf der Unterseite der biomedizinischen Elektrodenbaugruppe, die für den Hautkontakt vorgesehen ist, überlappt,
wobei die genannte Trägerkonstruktion (109, 112) dünner als das Gel ist, so dass das Gel vorsichtig durch die Trägerkonstruktion auf die Haut gedrückt wird, wodurch ein guter Kontakt zwischen Hydrogel und Haut sichergestellt wird.

2. Biomedizinische Elektrodenbaugruppe zur längeren Anwendung nach Anspruch 1 mit einem elektromechanischen Konnektor (102), der elektrisch mit den genannten Elektroden (108) verbunden ist, zur Verbindung mit einer Überwachungsvorrichtung.

3. Biomedizinische Elektrodenbaugruppe zur längeren Anwendung nach Anspruch 2, wobei der elektromechanische Konnektor (102) über mindestens einen leitenden Pfad pro Elektrode elektrisch mit den genannten Elektroden (108) verbunden ist, wobei der leitende Pfad von einer entsprechenden Elektrode zu dem genannten elektromechanischen Konnektor verläuft.

4. Biomedizinische Elektrodenbaugruppe zur längeren Anwendung nach einem der Ansprüche 1 bis 3, wobei die genannte Trägerkonstruktion (109, 112) Folgendes umfasst:
ein Trägersubstrat (109), das im Wesentlichen seitlich in Bezug auf die Gelpads angeordnet ist, die Gelpads umgibt und den Raum zwischen benachbarten Gelpads auffüllt,
mindestens eine Haltedichtung (112), die an einer Unterseite des genannten Trägersubstrats gegenüber einer dem Konnektor zugewandten Seite hiervon angebracht ist, wobei die Haltedichtung einen Außenumfang von jedem der Gelpads überlappt.

5. Biomedizinische Elektrodenbaugruppe zur längeren Anwendung nach einem der Ansprüche 1 bis 3, wobei die genannte Trägerkonstruktion (109, 112) eine Haltedichtung (112) umfasst, die im Wesentlichen seitlich in Bezug auf die Gelpads (111) angeordnet ist, die Gelpads umgibt, den Raum zwischen benachbarten Gelpads auffüllt und einen unteren Außenumfang von jedem der Gelpads überlappt.

6. Biomedizinische Elektrodenbaugruppe zur längeren Anwendung nach Anspruch 5, wobei die genannte Haltedichtung (112) Folgendes umfasst:
ein Substrat, das höchstens so dick ist wie die Hydrogelpads, im Wesentlichen seitlich in Bezug auf die Gelpads angeordnet ist, die Gelpads umgibt und den Raum zwischen benachbarten Gelpads auffüllt,
mindestens eine zusätzliche dünne Schicht, die an der Unterseite der genannten Haltedichtung gegenüber einer dem Konnektor zugewandten Seite hiervon angebracht ist, wobei diese zusätzliche dünne Schicht einen Außenumfang von jedem der Gelpads überlappt.

7. Biomedizinische Elektrodenbaugruppe zur längeren Anwendung nach einem der Ansprüche 1 bis 6, wobei die genannten Gelpads (111) ein ausgehärtetes Hydrogel umfassen.

8. Biomedizinische Elektrodenbaugruppe zur längeren Anwendung nach Anspruch 7, wobei das ausgehärtete Hydrogel ein aus einer vorab ausgehärteten Folie herausgeschnittenes Stück ist oder ein Teil, das vor Ort gegossen und ausgehärtet wurde.

9. Biomedizinische Elektrodenbaugruppe zur längeren Anwendung nach einem der Ansprüche 1 bis 8, wobei die genannte Trägerkonstruktion (109, 112) Schaumstoff mit dielektrischen Eigenschaften umfasst.

10. Biomedizinische Elektrodenbaugruppe nach einem der Ansprüche 1 bis 9, wobei die genannten leitenden Pfade durch eine Schaltungsschicht (106) verlaufen, die auf der einen Seite an den elektromechanischen Konnektor (102) und auf der genannten anderen Seite an die genannte Trägerkonstruktion (109, 112) gebondet ist

11. Medizinisches Überwachungs- und/oder Therapieabgabegerät mit einer biomedizinischen Elektrodenbaugruppe (100) zur längeren Anwendung nach einem der Ansprüche 1 bis 10.

12. Verfahren zum Anwenden der biomedizinischen Elektrodenbaugruppe (100) nach einem der Ansprüche 1 bis 11, wobei das Verfahren die folgenden Schritte umfasst:
Schaffen der genannten biomedizinischen Elektrodenbaugruppe (100), und
Befestigen der genannten biomedizinischen Elektrodenbaugruppe (100) an einem Patienten.

13. Verfahren nach Anspruch 12, weiterhin mit dem Schritt des Entfernens einer Schutzfolie von der Unterseite der genannten Trägerkonstruktion (109, 112), bevor die genannte biomedizinische Elektrodenbaugruppe (100) befestigt wird.

## Revendications

1. Ensemble d'électrode biomédicale à port prolongé (100), comprenant :
au moins deux plots de gel conducteur (111) espacés l'un par rapport à l'autre,
des électrodes (108) en contact avec les plots de gel (111),
une construction de support (109, 112) disposée sensiblement latéralement par rapport aux plots de gel (111), entourant les plots de gel, remplissant l'espace entre les plots de gel voisins et chevauchant un périmètre extérieur de chacun des plots de gel, sur le côté inférieur de l'ensemble d'électrode biomédicale qui est censé être en contact avec la peau,
dans lequel ladite construction de support (108, 112) est plus mince que le gel de telle sorte que le gel est poussé doucement à travers la construction de support sur la peau en garantissant un bon hydrogel au contact de la peau.

2. Ensemble d'électrode biomédicale à port prolongé selon la revendication 1, comprenant un connecteur électromécanique (102) connecté électriquement auxdites électrodes (108) pour connexion avec un dispositif de surveillance.

3. Ensemble d'électrode biomédicale à port prolongé selon la revendication 2, dans lequel le connecteur électromécanique (102) est connecté électriquement auxdites électrodes (108) via au moins un cheminement conducteur par électrode, chaque cheminement conducteur s'étendant d'une électrode correspondante audit connecteur électromécanique.

4. Ensemble d'électrode biomédicale à port prolongé selon l'une quelconque des revendications 1 à 3, dans lequel ladite construction de support (109, 112) comprend :
un substrat de support (109) disposé sensiblement latéralement par rapport aux plots de gel, entourant les plots de gel et remplissant l'espace entre les plots de gel voisins,
au moins un joint de rétention (112) fixé à une face inférieure dudit substrat de support opposé à un côté de celui-ci faisant face au connecteur, le joint de rétention chevauchant un périmètre externe de chacun des plots de gel.

5. Ensemble d'électrode biomédicale à port prolongé selon l'une quelconque des revendications 1 à 3, dans lequel ladite construction de support (109, 112) comprend un joint de rétention (112) disposé sensiblement latéralement par rapport aux plots de gel (111), entourant les plots de gel, remplissant l'espace entre les plots de gel voisins et chevauchant un périmètre externe inférieur de chacun des plots de gel.

6. Ensemble électrode biomédicale à port prolongé selon la revendication 5, dans lequel ledit joint de rétention (112) comprend :
un substrat au maximum aussi épais que les plots d'hydrogel disposés sensiblement latéralement par rapport aux plots de gel, entourant les plots de gel et remplissant l'espace entre les plots de gel voisins,
au moins une couche mince supplémentaire fixée à une face inférieure dudit joint de rétention opposée à un côté de celui-ci faisant face au connecteur, cette couche mince supplémentaire chevauchant un périmètre externe de chacun des plots de gel.

7. Ensemble d'électrode biomédicale à port prolongé selon l'une quelconque des revendications 1 à 6, dans lequel lesdits plots de gel (111) comprennent un hydrogel durci.

8. Ensemble d'électrode biomédicale à port prolongé selon la revendication 7, dans lequel l'hydrogel durci est un morceau découpé dans une feuille prédurcie ou est un élément qui est coulé et durci sur place.

9. Ensemble d'électrode biomédicale à port prolongé selon l'une quelconque des revendications 1 à 8, dans lequel ladite construction de support (109, 112) comprend de la mousse ayant des propriétés diélectriques.

10. Ensemble d'électrode biomédicale à port prolongé selon l'une quelconque des revendications 1 à 9, dans lequel lesdits cheminements conducteurs sont passés à travers une couche de circuit (106) liée au connecteur électromécanique (102) sur un côté et à ladite construction de support (109, 112) sur ledit autre côté.

11. Dispositif de surveillance médicale et/ou d'administration de thérapie comprenant un ensemble d'électrode biomédicale à port prolongé (100) selon l'une quelconque des revendications 1 à 10.

12. Procédé pour l'application de l'ensemble d'électrode biomédicale (100) selon l'une quelconque des revendications 1 à 11, le procédé comprenant les étapes consistant à :
fournir ledit ensemble d'électrode biomédicale (100), et
fixer ledit ensemble d'électrode biomédicale (100) à un patient.

13. Procédé selon la revendication 12, comprenant en outre l'étape consistant à retirer une feuille de protection de la face inférieure de ladite construction de support (109, 112) avant de fixer ledit ensemble d'électrode biomédicale (100).
